# EUROPÄISCHE PATENTANMELDUNG

(11) **EP 2 402 042 A2**
(43) Veröffentlichungstag der Anmeldung: **04.01.2012**
(21) Anmeldenummer: 11004744.6
(22) Anmeldetag: 10.06.2011
(51) Int. Cl.: A61L 27/30, A61L 27/32

(54) **Verfahren und Vorrichtung zur knochenwachstumsfördernden Beschichtung**

(30) Priorität: 29.06.2010 DE 102010025533
(71) Anmelder: Heraeus Medical GmbH, 61273 Wehrheim (DE)
(72) Erfinder: Vogt, Sebastian, 99092 Erfurt (DE); Büchner, Hubert, 90491 Nürnberg (DE)
(74) Vertreter: Panten, Kirsten

(57) **Zusammenfassung**

Die Erfindung bezieht sich auf ein Verfahren zur Beschichtung eines medizinischen Implantats, mit dem ein Calciumsalz auf einfache Weise ohne kostenintensive Beschichtungsapparaturen bei geringem Zeitaufwand auf die Oberfläche eines medizinischen Implantats aufgebracht werden kann.

Dementsprechend wird ein Verfahren zur Verfügung gestellt, bei dem man
(i) ein medizinisches Implantat bereitstellt, das wenigstens eine zu beschichtende Oberfläche aufweist,
(ii) einen Körper, der wenigstens an einer seiner Oberflächen wenigstens ein Calciumsalz mit einer Härte nach Mohs von nicht mehr als 5,5 aufweist, bereitstellt, und
(iii) den Körper mit der das Calciumsalz enthaltenden Oberfläche auf die wenigstens eine zu beochiohtondo Oberfläche des medizinischen Implantats reibt, um diese Oberfläche des medizinischen Implantats mit wenigstens dem Calciumsalz zu beschichten.

## Beschreibung

Die Erfindung betrifft ein Verfahren zur Beschichtung eines medizinischen Implantats, die Verwendung eines Körpers zur Beschichtung eines medizinischen Implantats und einen Stift zur Beschichtung eines medizinischen Implantats.

Medizinische Implantate werden seit langem in der Medizin mit der Aufgabe eingesetzt, Körperfunktionen zu unterstützen oder zu ersetzen. Insbesondere defekte Körpergelenke werden zunehmend mit großem Erfolg durch Gelenkimplantate ersetzt, was oft mit einer deutlichen Steigerung der Lebensqualitat des betroffenen Palienten einhergeht.

Zum Ersatz von Hüft-, Knie-und Schultergelenken kommen gegenwärtig neben zementierten Gelenkendoprothesen auch zunehmend nicht zementierte Gelenkendoprothesen zur Anwendung. Nicht zementierte Gelenkendoprothesen sind dabei im Allgemeinen aus Titanlegierungen gefertigt und besitzen zumeist eine aufgeraute (oft gesandstrahlte) oder eine strukturierte poröse Oberfläche, um ein Anwachsen des Knochengewebes zu verbessern.

Aus dem Stand der Technik sind Versuche bekannt, die Kompatibilität der Implantatoberflächen gegenüber dem Knochengewebe zu verbessern. Dabei sollte stets eine lasttragende Bulk-Phase vorwiegend die geforderten biomechanischen Eigenschaften einbringen (Strukturkompatibilität) und die Oberflächenphase die Kompatibilität (Oberflächenkompatibilität) zum angrenzenden Knochengewebe gewährleisten (WINTERMANTEL: Biokompatible Werkstoffe und Bauweisen: Implantate für die Medizin und Umwelt. Springer Verlag, Berlin, Heidelberg, New York, 1996). Die Konzeption bestand darin, solche Oberflächenstrukturen zu erzeugen, die die mineralische Phase des Knochengewebes nachbilden. Die mineralische Phase des humanen Knochengewebes wird durch einen Carbonat-Apatit/Hydroxylapatit gebildet. Deshalb lag in der Entwicklung von Calciumphosphat-Schichten der Hauptschwerpunkt zur Verbesserung der Oberflächenkompatibilität von Implantaten.

Im Bereich des Knochengewebekontaktes (Hüft-, Knie-, Schulter-Gelenkendoprothesen) wurde mit unterschiedlichsten Verfahren (thermische Spritzverfahren, elektrochemische Abscheidung, Sol-Gel-Technologien, Ionenstrahlsputtern, Laserablation) versucht, eine Verbesserung der Oberflächenkompatibilität zu erreichen. Es konnten sich bisher industriell nur das Plasmaspritzverfahren (DE GROOT, KLEIN, WOLKE: Plasma-sprayed coatings of calcium phosphate. CRC Press, Boca Raton, Ann Arbor, Boston, 1990; DE GROOT, KLEIN, WOLKE: Chemistry of calcium phosphate bioceramics. CRC Handbook of bioactive ceramics, 2 (1996) 3-16; W02009062671) und die elektrochemische Abscheidung von Calciumphosphat-Schichten (BAN, MARUNO: Morphology and microstructure of electrochemically deposited calcium phosphates in a modified simulated body fluid. Biomaterials, 19 (1998) 1245-1253; DE4431862; WO200914704 CN101485901; CN101406711; EP2037980; US2006134160; WO2004098436; WO2004024201; EP1264606; EP0774982; EP0232791) durchsetzen.

Klinische Langzeituntersuchungen zeigten jedoch, dass die als langzeitstabil geltenden plasmagespritzten Calciumphosphat-Schichten im biologischen Umgebungsmilieu einer partiellen Degradation unterliegen (WHEELER: Eight-year clinical retrospective study of titanium plasma-sprayed and hydroxyapatite-coated cylinder implants. International Journal of Oral and Maxillofacial Implants, 11,3 (1996) 340-350. OSHBORN: Die biologische Leistung der Hydroxylapatitkeramik-Beschichtung auf dem Femurschaft einer Titanendoprothese-erste histologische Auswertung eines Humanexplantats. Biomedizinische Technik, 32 (1987) 177-183.). Dabei kommt es einerseits zu Phasenänderungen an der Grenzfläche zum Knochengewebe; andererseits führt dieser Prozess zum Abkapseln und/oder Abplatzen von vorwiegend kristallinen Schichtbestandteilen (Partikeln).

In Untersuchungen von Cooley (COOLEY, VAN DELLEN, BURGESS, WINDELER: The advantages of coated titanium implants prepared by radiofrequency sputtering from hydroxyapatite. J. Prosthet. Dent., 67 (1992) 93-100.) und Maxian (MAXIAN, ZAWADSKI, DUNN: Effect of CaP coating resorption and surgical fit on the bone/implant interface. Journal of Biomedical Material Research. 28 (1994) 1311-1319.) wurde die hohe Effizienz von vollständio dearadierbaren. bioaktiven Schichten, die mittels elektrochemischer Verfahren auf metallischen Grundkörpern aufgebracht wurden, nachgewiesen. Die Auswertung von tierexperimentellen und klinischen Studien führte zu der Schlussfolgerung, dass trotz der schnellen und vollständigen Degradation von hochlöslictien Calciumphosphat-Schichten ein sicheres Knochenanwachsverhalten an die Implantatoberfläche gegeben ist.

Es kann daher festgestellt werden, dass die schnelllöslichen Calciumphosphat-Schichten zu klinisch guten Resultaten führen können. Zur Verbesserung der Kompatibilität von Implantatoberflächen gegenüber dem Knochengewebe ist offensichtlich keine langzeitstabile Beschichtung auf den Implantatoberflächen erforderlich.

Nachteilig ist jedoch bei allen bisher üblichen elektrochemischen Abscheidungsverfahren, dass ein erheblicher apparativer und zeitlicher Aufwand notwendig ist, um diese Calciumphosphat-Schichten auf diese medizinischen Implantate aufzubringen.

Es besteht daher Bedarf an einem einfachen, kostengünstigen und schnell anwendbaren Verfahren, mit dem Oberflächen von medizinischen Implantaten mit Beschichtungen versehen werden können, die das Knochenwachstum fördern.

Der Erfindung lag daher die Aufgabe zugrunde, ein Verfahren zur Beschichtung eines medizinischen Implantats bereitzustellen, mit dem ein Calciumsalz auf einfache Weise ohne kostenintensive Beschichtungsapparaturen bei geringem Zeitaufwand auf die Oberfläche eines medizinischen Implantats aufgebracht werden kann. Dieses Verfahren soll insbesondere unmittelbar vor der Implantation im Operationssaal angewendet werden können. Ferner soll dieses Verfahren die Herstellung einer das Knochenwachstum stimulierenden und zugleich hämostyptisch wirkenden Beschichtung von nicht zementierten Gelenkendoprothesen ermöglichen.

Ebenso soll eine Vorrichtung zur Verfügung gestellt werden, mit der das erfindungsgemäße Verfahren ausführbar ist.

Die erfindungsgemäße Aufgabe wird gelöst durch ein Verfahren zur Beschichtung eines medizinischen Implantats, bei dem man
(i) ein medizinisches Implantat bereitstellt, das wenigstens eine zu beschichtende Oberfläche aufweist,
(ii) einen Körper, der wenigstens an einer seiner Oberflächen wenigstens ein Calciumsalz mit einer Härte nach Mohs von nicht mehr als 5,5 aufweist, bereitstellt, und
(iii) den Körper mit der das Calciumsalz enthaltenden Oberfläche auf die wenigstens eine zu beschichtende Oberfläche des medizinischen Implantats reibt, um diese Oberfläche des medizinischen Implantats mit wenigstens dem Calciumsalz zu beschichten.

Zur Durchführung dieses Verfahrens kann ein Stift zur Beschichtung eines medizinischen Implantats verwendet werden, der einen Hohlkörper und einen in dem Hohlkörper bewegbar und aus dem Hohlkörper wenigstens teilweise ausführbar angeordneten Körper, der ein Calciumsalz mit einer Härte nach Mohs von nicht mehr als 5,5 und einen pharmazeutischen Wirkstoff umfasst, aufweist.

Der Erfindung liegt die Erkenntnis zugrunde, dass medizinische Implantate, insbesondere nicht zementierte Gelenkendoprothesen, üblicherweise eine raue oder strukturierte Oberfläche besitzen, um eine gute Integration des Knochengewebes zu erreichen. Es wurde überraschenderweise gefunden, dass medizinische Implantate auf einfache Weise mit Calciumsalzen, die eine Härte nach Mohs von nicht mehr als 5,5 aufweisen, beschichtet werden können, wenn diese Calciumsalze auf diese Oberflächen der medizinischen Implantate gerieben werden. Beim Reiben erfolgt ein Abtrag des weichen Calciumsalzes, das in der rauen oder strukturierten Oberfläche des medizinischen Implantats verbleibt. Da es offensichtlich ausreicht, wie die Ergebnisse mit auflösenden Calciumphosphat-Schichten zeigten, dass eine Beschichtung nur temporär auf den Implantatoberflächen vorhanden sein muss, ist die Verwendung von degradierbaren, weichen Beschichtungsmaterialien möglich.

Die Erfindung ermöglicht somit die Beschichtung eines medizinischen Implantats mit wenigstens einem Calciumsalz durch den Anwender unmittelbar vor der Implantation im Operationssaal. Zur Verwirklichung des erfindungsgemäßen Beschichtungsverfahrens sind mithin keine besonderen logistischen Voraussetzungen zu schaffen, so dass beispielsweise Knieendoprothesen aus verschiedenen Materialien mit unterschiedlichen Formen und von verschiedenen Herstellern ohne weitere technische Hilfsmittel mit wenigstens einem Calciumsalz beschichtet werden können. Neben dem wenigstens einem Calciumsalz kann die zu beschichtende Oberfläche mittels dem erfindungsgemäßen Verfahren auch mit einem oder mehreren weiteren Substanzen beschichtet werden.

Erfindungsgemäß wird somit ein Verfahren zur Beschichtung eines medizinischen Implantats bereitgestellt. Insbesondere wird ein Verfahren zur Herotollung einer das Knochenwastum fördernden Beschichtung auf der Oberfläche eines medizinischen Implantats bereitgestellt. Mit diesem Verfahren gelingt ebenfalls die Herstellung einer hämostyptisch wirksamen Beschichtung auf der Oberfläche eines medizinischen Implantats. Gegenstand dieses Verfahrens ist ferner die Beschichtung der Oberfläche eines medizinischen Implantats mit einem Calciumsalz, das eine Härte nach Mohs von nicht mehr als 5,5 aufweist.

Dieses Verfahren wird erfindungsgemäß außerhalb des menschlichen oder tierischen Organismus durchgeführt.

Hierzu wird zunächst ein medizinisches Implantat bereitgestellt, das wenigstens eine zu beschichtende Oberfläche aufweist.

Im Rahmen der Erfindung ist unter Beschichten eines medizinischen Implantats das Bedecken von wenigstens einem Teil wenigstens einer Oberfläche des Implantats zu verstehen. Gemäß einer bevorzugten Ausführungsform ist unter Beschichten eines medizinischen Implantats das Bedecken von wenigstens 30 %, mehr bevorzugt wenigstens 40 %, noch mehr bevorzugt wenigstens 50 %, besonders bevorzugt wenigstens 60% und ganz besonders bevorzugt wenigstens 70 % wenigstens einer Oberfläche des Implantats zu verstehen.

Unter den Begriff medizinisches Implantat fallen erfindungsgemäß Materialien und Vorrichtungcn, die im Zuge eines chirurgischen Eingriffes mindestens teilweise im Körperinneren eingebracht werden. Diese Implantate können im Kontakt zum Knochen und anderen Elementen des Stütz- und Bewegungsapparates sowie auch in Kontakt mit Blut oder Bindegewebe stehen.

Gemäß einer bevorzugten Ausführungsform handelt es sich bei dem medizinischen Implantat um eine Endoprothese. Unter Endoprothesen sind Implantate zu verstehen, die dauerhaft im Körper verbleiben und einen Körperteil ganz oder teilweise ersetzen.

Gemäß einer besonders bevorzugten Ausführungsform handelt es sich bei dem medizinischen Implantat um eine Gelenkendoprothese. Als beispielhafte Gelenkendoprothesen sind Kniegelenkendoprothesen und Hüftgelenkendoprothesen zu nennen. Vorzugsweise handelt es sich bei den Gelenkendoprothesen um nicht zementierte Gelenkendoprothesen.

Diese medizinischen Implantate können aus verschiedenen Materialien gefertigt sein. Gemäß einer bevorzugten Ausführungsform umfasst das medizinische Implantat Titan oder Titanlegierungen oder besteht im Wesentlichen daraus.

Erfindungsgemäß kann es bevorzugt sein, dass wenigstens eine Oberfläche rau ist. Gemäß einer bevorzugten Ausführungsform weist wenigstens eine Oberfläche des medizinischen Implantats eine mittlere Rauheit Rₐ von wenigstens 0,5 µm, besonders bevorzugt von wenigstens 0,75 µm und ganz besonders bevorzugt von wenigstens 1,0 µm auf. Die mittlere Rauheit Rₐ gibt den mittleren Abstand eines Messpunktes - auf der Oberfläche - zur Mittellinie an. Die Mittellinie schneidet innerhalb der Bezugsstrecke das wirkliche Profil so, dass die Summe der Profilabweichungen (bezogen auf die Mittellinie) minimal wird. Demnach entspricht die mittlere Rauheit Rₐ dem arithmetischen Mittel der Abweichung von der Mittellinie. Zur Messung der Rauheit wird erfindungsgemäß eine profilbasierte Methode unter Verwendung eines Tastschnittgerätes bevorzugt. Bei der wenigstens einen Oberfläche mit einer mittleren Rauheit Rₐ von wenigstens 0,5 µm, besonders bevorzugt von wenigstens 0,75 µm und ganz besonders bevorzugt von wenigstens 1,0 µm, handelt es sich vorzugsweise um die zu beschichtende Oberfläche des medizinischen Implantats. Überraschenderweise hat sich herausgestellt, dass sich raue Implantatoberflächen besonders leicht und stabil mit Calciumsalzen beschichten lassen. Dies könnte darauf zurückzuführen sein, dass beim Reiben des das wenigstens eine Calciumsalz enthaltenden Körpers auf der rauen Oberfläche des medizinischen Implantats ein das Calciumsalz enthaltender Abrieb erzeugt wird, der zwischen den das Profil der Oberfläche des medizinischen Implantats definierende Vertiefungen abgeschieden wird.

Die zu beschichtende Oberfläche des medizinischen Implantats weist ferner vorzugsweise eine Härte nach Mohs von wenigstens 3,5, mehr bevorzugt von wenigstens 4,0, noch mehr bevorzugt von wenigstens 4,5 und ganz besonders bevorzugt von wenigstens 5 auf. Es hat sich gezeigt, dass der Abrieb des Calciumsalzes mit einer Härte nach Mohs von nicht mehr als 5,5 auf der Oberfläche des medizinischen Implantats umso größer ist, je größer die Härte nach Mohs der zu beschichtenden Oberfläche des medizinischen Implantats ist. Gemäß einer besonders bevorzugten Ausführungsform weist die zu beschichtende Oberfläche des medizinischen Implantats eine Härte nach Mohs auf, die größer ist als die Härte nach Mohs des Calciumsalzes. Gemäß einer weiteren besonders bevorzugten Ausführungsform weist die zu beschichtende Oberfläche des medizinischen Implantats eine Härte nach Mohs auf, die größer ist als die Härte nach Mohs der das Calciumsalz aufweisenden Oberfläche Körpers.

Erfindungsgemäß wird zur Beschichtung des medizinischen Implantats ein Körper verwendet, der wenigstens an einer seiner Oberflächen wenigstens ein Calciumsalz mit einer Härte nach Mohs von nicht mehr als 5,5, mehr bevorzugt von nicht mehr als 5,0, noch mehr bevorzugt von nicht mehr als 4,5, besonders bevorzugt von nicht mehr als 4,0 und ganz besonders bevorzugt von nicht mehr als 3,5 aufweist.

Im Rahmen der Erfindung ist unter Körper jeder Gegenstand ohne Einschränkung hinsichtlich seiner Geometrie zu verstehen. Insbesondere kann es sich bei dem Körper um einen Zylinder handeln. Gemäß einer bevorzugten Ausführungsform ist der Körper mit wenigstens drei Fingern einer Hand greifbar. Gemäß einer weiteren bevorzugten Ausführungsform weist der Körper ein Volumen von wenigstens 50 mm³, mehr bevorzugt ein Volumen von wenigstens 100 mm³, noch mehr bevorzugt ein Volumen von wenigstens 250 mm³, besonders bevorzugt ein Volumen von wenigstens 330 mm³ und ganz besonders bevorzugt ein Volumen von wenigstens 15000 mm³.

Der Körper weist wenigstens an einer seiner Oberflächen wenigstens ein Calciumsalz mit einer Härte nach Mohs von nicht mehr als 5,5, mehr bevorzugt von nicht mehr als 5,0, noch mehr bevorzugt von nicht mehr als 4,5, besonders bevorzugt von nicht mehr als 4,0, ganz besonders bevorzugt von nicht mehr als 3,5 und insbesondere von nicht mehr als 3,1 auf. Vorzugsweise weisen alle Oberflächen des Körpers wenigstens ein Calciumsalz mit einer Härte nach Mohs von nicht mehr als 5,5, mehr bevorzugt von nicht mehr als 5,0, noch mehr bevorzugt von nicht mehr als 4,5, besonders bevorzugt von nicht mehr als 4,0, ganz besonders bevorzugt von nicht mehr als 3,5 und insbesondere von nicht mehr als 3,1 auf. Gemäß einer bevorzugten Ausführungsform weist nicht nur wenigstens eine Oberfläche des Körpers ein Calciumsalz mit einer Härte nach Mohs von nicht mehr als 5,5, mehr bevorzugt von nicht mehr als 5,0, noch mehr bevorzugt von nicht mehr als 4,5, besonders bevorzugt von nicht mehr als 4,0, ganz besonders bevorzugt von nicht mehr als 3,5 und insbesondere von nicht mehr als 3,1 auf. Insbesondere ist es bevorzugt, dass das wenigstens eine Calciumsalz mit einer Härte nach Mohs von nicht mehr als 5,5, mehr bevorzugt von nicht mehr als 5,0, noch mehr bevorzugt von nicht mehr als 4,5, besonders bevorzugt von nicht mehr als 4,0, ganz besonders bevorzugt von nicht mehr als 3,5 und insbesondere von nicht mehr als 3,1 homogen oder heterogen in dem Körper verteilt ist.

Das wenigstens an einer Oberfläche des Körpers vorhandene Calciumsalz ist vorzugsweise biokompatibel.

Gemäß einer bevorzugten Ausführungsform weist wenigstens eine Oberfläche des Körpers, insbesondere die Oberfläche des Körpers, die auf die zu beschichtende Oberfläche des Implantats gerieben wird, eine Härte nach Mohs von nicht mehr als 5,5, mehr bevorzugt von nicht mehr als 5,0, noch mehr bevorzugt von nicht mehr als 4,5, besonders bevorzugt von nicht mehr als 4,0, ganz besonders bevorzugt von nicht mehr als 3,5 und insbesondere von nicht mehr als 3,1 auf.

Ferner weist der Körper vorzugsweise eine Porosität im Bereich von 0-70 Volumenprozent auf. Unter Porosität ist erfindungsgemäß das Verhältnis von Hohlraumvolumen zu Gesamtvolumen des Körpers zu verstehen.

Gemäß einer Ausführungsform kann es bevorzugt sein, dass die Porosität des Körpers im Bereich von 0 -15 %, mehr bevorzugt im Bereich von 0 -10 % und besonders bevorzugt im Bereich von 0 - 5 % liegt. In diesen Fällen ist es bevorzugt, dass der Körper neben dem wenigstens einen Calciumsalz weitere Inhaltsstoffe, insbesondere die nachstehend aufgeführten Inhaltsstoffe (bspw. wenigstens einen pharmazeutischen Wirkstoff), enthält. Wenn der Anwender die Gegenwart dieser Inhaltsstoffe in der Implantatbeschichtung für erforderlich enthält, lässt sich die Oberfläche des medizinischen Implantats daher mittels dieses Körpers direkt beschichten, ohne dass die Zugabe dieser Inhaltsstoffe zu dem Körper durch den Anwender nötig wäre. Die niedrige Porosität dieses Körpers hat sich als besonders geeignet erwiesen, um einen hohen Abrieb des wenigstens einen Calciumsalzes auf der Oberfläche des medizinischen Implantats zu erzeugen.

Gemäß einer weiteren Ausführungsform kann es bevorzugt sein, dass die Porosität des Körpers im Bereich von 10 - 70 %, mehr bevorzugt im Bereich von 20 - 70 % und besonders bevorzugt im Bereich von 30 - 70 % liegt. In diesen Fällen kann es vorgesehen sein, dem Körper unmittelbar vor der Anwendung weitere Inhaltsstoffe, insbesondere die nachstehend aufgeführten Inhaltsstoffe (bspw. wenigstens einen pharmazeutischen Wirkstoff), zuzusetzen. Wenn der Anwender die Gegenwart dieser Inhaltsstoffe in der Implantatbeschichtung für erforderlich enthält, können diese Inhaltsstoffe dem Körper zugesetzt werden und verbleiben aufgrund der angegebenen Porosität im Körper enthalten. Beispielsweise ist es möglich, die Körper vor der Anwendung mit einer Lösung zu tränken, die wenigstens einen weiteren Inhaltsstoff, bspw. einen pharmazeutischen Wirkstoff, enthält. Aufgrund der bezeichneten Porosität ist der Körper in der Lage, diesen Inhaltsstoff aufzunehmen. Dadurch wird üblicherweise die Porosität des Körpers abgesenkt, wodurch sich die Oberfläche des medizinischen Implantats mittels dieses Körpers einfach und sicher beschichten lässt. Diese Ausführungsform ermöglicht somit eine Präparation des Körpers zur Herstellung einer weitere Inhaltsstoffe enthaltenden Beschichtung, die auf die spezifischen Bedürfnisse des Implantatempfängers angepasst sind.

Gemäß einer weiteren bevorzugten Ausführungsform weist der Körper einen Wasseranteil im Bereich von 0-70 Gewichtsprozent auf. Der Wasseranteil kann beispielsweise auf im Körper eingeschlossenes Wasser und/oder auf mit den Calciumsalzen verbundenes Kristallwasser zurückzuführen sein.

Dabei kann es einerseits bevorzugt sein, dass der Körper einen Wasseranteil im Bereich von 0 - 20 Gewichtsprozent, mehr bevorzugt im Bereich von 0 -10 Gewichtsprozent und besonders bevorzugt im Bereich von 0 - 5 Gewichtsprozent aufweist. Dieser Wasseranteil kann zum einen gegeben sein, wenn der Körper bereits alle gewünschten Inhaltsstoffe enthält und direkt für die Herstellung der Beschichtung des medizinischen Implantats eingesetzt werden kann ("ready-touse"). Zum anderen kann dieser Wasseranteil gegeben sein, wenn der Körper noch nicht alle Inhaltsstoffe enthält, sondern dem Körper weitere Inhaltsstoffe unmittelbar vor der Anwendung zugegeben werden sollen. In diesem Fall ist der Körper aufgrund des geringen Wasseranteils in der Lage, Lösungen dieser weiteren Inhaltsstoffe aufzunehmen. Körper mit diesem geringen Wasseranteil können somit auf einfache Weise, bspw durch Tränken mit einer Lösung des wenigstens einen weiteren Inhaltsstoffs, präpariert werden, um zu einem Körper gelangen, mit dem sich eine Implantatbeschichtung erzeugen lässt, die auf die speziellen Bedürfnisse des Implantatempfängers angepasst sind.

Andererseits kann es bevorzugt sein, dass der Körper einen Wasseranteil im Bereich von 20 - 70 Gewichtsprozent, mehr bevorzugt im Bereich von 25 - 65 Gewichtsprozent und besonders bevorzugt im Bereich von 30 - 60 Gewichtsprozent aufweist. Dieser Wasseranteil wird insbesondere in Fällen erreicht, in denen dem Körper unmittelbar vor der Anwendung weitere Inhaltsstoffe, bspw. durch Tränken mit einer wässrigen Lösung des wenigstens einen weiteren Inhaltsstoffs, zugesetzt werden und eine Trocknung des Körpers unterbleibt.

Gemäß noch einer weiteren bevorzugten Ausführungsform weist das Calciumsalz eine Löslichkeit in Wasser bei einer Temperatur von 25°C von wenigstens 2 g/l. mehr bevorzugt von wenigstens 5 g/l und noch mehr bevorzugt von wenigstens 10 g/l auf. Calciumsalze, die eine hohe Wasserlöslichkeit aufweisen und Calcium freisetzen, sind erfingdungsgemäß bevorzugt, da sie als Blutgerinnungsfaktor IV ein Hämostyptikum darstellen und nach der Implantation die Blutgerinnungskaskade aktivieren. Wird so eine Blutgerinnung durch auf der Oberfläche von medizinischen Implantaten befindliche Stoffen ausgelöst, führt dies unmittelbar nach der Implantation zur Ausbildung einer Fibrinschicht, die das Anwachsen des medizinischen Implantats am Knochen begünstigt.

Erfindungsgemäß kann es bevorzugt sein, wenn es sich bei den Im Körper enthaltenen Calciumsalzen um anorganische Calciumsalze handelt. Andererseits ist es möglich, dass in dem Körper neben anorganischen Calciumsalzen auch organische Calciumsalze enthalten sind.

Vorzugsweise beträgt der Anteil an Celciumsalzen in dem Körper wenigstens 50 Gewichtsprozent, mehr bevorzugt wenigstens 60 Gewichtsprozent, noch mehr bevorzugt wenigstens 70 Gewichtsprozent, besonders bevorzugt wenigstens 75 Gewichtsprozent, ganz besonders bevorzugt wenigstens 80 Gewichtsprozent und insbesondere wenigstens 85 Gewichtsprozent, bezogen auf das Gewicht des Körpers. Beispielsweise können in dem Körper 50 - 100 Gewichtsprozent, mehr bevorzugt 60 -100 Gewichtsprozent, noch mehr bevorzugt 70 - 100 Gewichtsprozent, besonders bevorzugt 75 -100 Gewichtsprozent, ganz besonders bevorzugt 80 - 100 Gewichtsprozent und insbesondere 85 -100 Gewichtsprozent Calciumsalze, bezogen auf das Gewicht des Körpers, enthalten sein. Ebenso können in dem Körper beispielsweise 50 - 90 Gewichtsprozent, mehr bevorzugt 50 - 85 Gewichtsprozent, noch mehr bevorzugt 55 - 85 Gewichtsprozent, besonders bevorzugt 60 - 85 Gewichtsprozent, ganz besonders bevorzugt 65 - 85 Gewichtsprozent und insbesondere 60 - 80 Gewichtsprozent Calciumsalze, bezogen auf das Gewicht des Körpers, enthalten sein.

Gemäß einer weiteren bevorzugten Ausführungsform ist das Calciumsalz aus der Gruppe ausgewählt, die aus Calciumsulfaten, Calciumphosphaten, Calciumhydrogenphosphaten und Calciumcarbonat besteht.

Erfindungsgemäß sind unter dem Begriff Calciumsalze sowohl kristallwasserhaltige Calciumsalze wie auch kristallwasserfreie Calciumsalze zu verstehen. So kann beispielsweise das Calciumsulfat aus der Gruppe ausgewählt sein, die aus kristallwasserhaltigem Calciumsulfat und kristallwasserfreiem Calciumsulfat besteht Dabei ist das kristallwasserhaltige Calciumsulfat vorzugeweise aus der Gruppe ausgewählt. die aus Calciumsulfat-Dihydrat und Calciumsulfat-Hemihydrat besteht Calcium-Dihydrat hat sich dabei als besonders vorteilhaft erwiesen, da es im ausgehärteten Zustand porös ist und daher Wasser oder wässrige Lösungen aufsaugen kann. Dadurch ist es bspw. auf einfache Weise möglich, den gebildeten Körper nachträglich mit wässrigen Lösungen von weiteren Inhaltsstoffen, wie zum Beispiel pharmazeutischen Wirkstoffen, zu tränken.

Gemäß einer besonders bevorzugten Ansführungsform ist das Calciumsulfat aus der Gruppe ausgewählt, die aus kristallwasserfreiem Calciumsulfat, Calciumsulfat-Dihydrat und Calciumsulfat-Hemihydrat besteht.

Gemäß einer weiteren besonders bevorzugten Ausführungsform ist das Calciumphosphat aus der Gruppe ausgewählt, die aus kristallwasserfreiem Calciumphosphat, alpha-Tricalciumphosphat (α-TCP) und beta-Tricalciumphosphat (β-TCP) besteht.

Gemäß noch einer weiteren besonders bevorzugten Ausführungsform ist das Calciumhydrogenphosphat aus der Gruppe ausgewählt, die aus Brushit und Monetit besteht.

Vorzugsweise enthält der Körper ferner eine Verbindung, die aus der Gruppe ausgewählt ist, die aus Fettsäuren, Fettsäureestern, Fettsäuresalzen, Polyethylenglykolen mit einer Molmasse von nicht mehr als 1000 g/mol und oligomeren Milchsäureestern mit einer Molmasse von weniger als 1000 g/mol besteht. Gemaß einer besonders bevorzugten Ausführungsform handelt es sich bei dieser Verbindung um einen Glycerintrifettsäureester.

Diese Verbindungen können beispielsweise dazu dienen, weitere Inhaltsstoffe, insbesondere weitere pharmazeutische Wirkstoffe, an den Körper zu binden. Ferner können diese Verbindungen als Bindemittel beim Herstellen der Körper dienen.

Gemäß einer weiteren Ausführungsform enthält der Körper einen pharmazeutischen Wirkstoff.

Dieser pharmazeutische Wirkstoff kann vorzugsweise aus der Gruppe ausgewählt sein, die aus Antibiotika, Antiseptika, Aniphlogistika, Steroide, Hormonen, Wachstumsfaktoren, Bisphosphonaten, Cytostatika, Genvektoren und Plasmiden besteht. Gemäß einer besonders bevorzugten Ausführungsform handelt es sich bei dem wenigstens einen pharmazeutischen Wirkstoff um ein Antibiotikum.

Das wenigstens eine Antibiotikum wird vorzugsweise ausgewählt aus den Gruppen der Aminoglykosid-Antibiotika, der Glykopeptid-Antibiotika, der Lincosamid-Antibiotika, der Chinolon-Antibiotika, der Oxazolidinon-Antibiotika, der Gyrasehemmer, der Carbapeneme, der cyclischen Lipopeptide, der Glycylcycline und der Peptidantibiotika.

Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid und Moxifloxacin besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem Fibroblast Growth Factor (FGF), dem Transforming Growth Factor (TGF), dem Platelet Derived Growth Factor (PDGF), dem Epidermalen Wachstumsfaktor (EGF), dem Vascular Endothelial Growth Factor (VEGF), insulinähnlichen Wachstumsfaktoren (IGF), dem Hepatocyte Growth Factor (HGF), dem Bone Morphogenic Protein (BMP), Interleukin-1B, Interleukin 8 und dem Nervenwachstumsfaktor besteht.

Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht.

Das wenigstens eine Bisphosphonat ist vorzugsweise aus der Gruppe ausgewählt, die aus Zoledronat und Aledronat besteht.

Bei dem Genvektor kann es sich um einen viralen Vektor oder einen Cosmid-Vektor handeln.

Der Anteil des pharmazeutischen Wirkstoffs an dem Körper ist nicht weiter eingeschränkt. Er kann beispielsweise im Bereich von 0,1-30 Gewichtsprozent, mehr bevorzugt im Bereich von 0,5-20 Gewichtsprozent und besonders bevorzugt im Bereich von 1 -15 Gewichtsprozent, bezogen auf das Gewicht des Körpers, liegen.

Der pharmazeutische Wirkstoff kann auf dem Körper als Beschichtung oder homogen in dem Körper verteilt vorliegen.

Der Körper kann ferner wenigstens einen Farbstoff enthalten. Bei dem Farbstoff handelt es sich besonders bevorzugt um einen Lebensmittelfarbstoff. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, eine Mischung aus E104 und E132. Durch die Gegenwart des Farbstoffs kann vom Anwender des Körpers während des erfindungsgemäßen Beschichtungsverfahrens leicht erkannt werden, welche Bereiche des medizinischen Implantats bereits beschichtet sind und welche nicht.

Es kann im Rahmen der Erfindung auch bevorzugt sein, dass der Körper wenigstens ein Salz enthält, das aus der Gruppe ausgewählt ist, die aus Magnesiumsalzen, Strontiumsalzen und Lithiumsalzen besteht. Ein bevorzugtes Salz ist beispielsweise Strontiumsulfat. Mit diesen Salzen lässt sich eine verbesserte Osteointegration der mittels dieses Körpers beschichteten medizinischen Implantate, insbesondere der Gelenkendoprothesen, erreichen.

Gemäß einer bevorzugten Ausführungsform enthält der Körper 70 - 90 Gewichtsprozent Calciumsulfat und 10-30 Gewichtsprozent Calciumcarbonat. Bei dem Calciumsulfat handelt es sich hierbei vorzugsweise um Calciumsulfat-Dihydrat.

Gemäß einer weiteren bevorzugten Ausführungsform enthält der Körper 40 - 90 Gewichtsprozent Calciumsulfat, 0,1-40 Gewichtsprozent Calciumcarbonat und 0,1-20 Gewichtsprozent Strontiumsulfat. Hier kann es bevorzugt sein, dass das Calciumsulfat Calciumsutfat-Dihydrat ist. Gemäß einer weiteren bevorzugten Ausführungsform enthält der Körper 20-90 Gewichtsprozent Calciumsulfat, 0,1-40 Gewichtsprozent Calciumcarbonat und 0,5-50 Gewichtsprozent eines pharmazeutischen Wirkstoffs.

Gemäß noch einer weiteren bevorzugten Ausführungsform enthält der Körper 70 - 73 Gewichtsprozent Calciumsulfat, 16 - 20 Gewichtsprozent Calciumcarbonat, 8-10 Gewichtsprozent Glycerintripalmitat und 1 - 2 Gewichtsprozent Gentamicinsulfat. Bei dem Calciumsalz handelt es sich vorzugsweise um Gaiciumsulfat-Dihydrat.

Der erfindungsgemäße Körper kann auf verschiedene Weisen hergestellt werden.

Gemäß einer bevorzugten Ausführungsform wird der Körper durch Pressung eines Pulvers hergestellt. Dieses Pulver weist vorzugsweise dieselben Bestandteile bevorzugt in derselben Zusammensetzung auf, wie dies hierin in Bezug auf den Körper beschrieben wird. Der Körper kann aus diesem Pulver durch einfaches Pressen, vorzugsweise mit Exzenterpressen, geformt werden. Durch Variation der Einpresstiefe kann dabei die Porosität und das Abriebverhalten des Körpers gesteuert werden. Als besonders vorteilhaft hat sich dabei der Zusatz von Bindemitteln, insbesondere Glycerintrifettsäureestern, erwiesen.

Gemäß einer weiteren bevorzugten Ausführungsform wird der Körper durch Gießen einer selbstaushärtenden Mischung hergestellt. Diese selbstaushärtende Mischung enthält vorzugsweise wenigstens ein Calciumsalz, bspw. Calciumsulfat-Hemihydrat, und Wasser. Vorzugsweise wird der Körper direkt im nachstehend beschriebenen Hohlkörper durch Eingießen der selbsthärtenden Mischung hergestellt. Gemäß dieser Ausführungsform können auf einfache Weise in Wasser lösliche oder suspendlerbare Bestandteile, so zum Beispiel pharmazeutische Wirkstoffe, in die selbsthärtende Mischung mit eingebracht werden. Diese Bestandteile werden folglich beim Aushärten der Mischung in dem Körper eingeschlossen. Die Härtung der Mischung erfolgt vorzugsweise durch die Anlagerung von Wasser an das in der Mischung enthaltene Calciumsalz. Handelt es sich bei dem Calciumsalz bspw. um Caiciumsulfat-Hemihydrat, so bildet sich bei dieser Wasseranlagerung Calciumsulfat-Dihydrat. Durch diese Anlagerung kommt es infolge der Ausbildung von verfilzten Kristallstrukturen zur Aushärtung der Mischung.

Unter Verwendung des hierin beschriebenen Körpers kann wenigstens eine Oberfläche eines medizinischen Implantats beschichtet werden. Dazu wird der Körper mit der das Calciumsalz enthaltenden Oberfläche auf die wenigstens eine zu beschichtende Oberfläche des medzinischen Implantats gerieben, um diese Oberfläche des medizinischen Implantats mit wenigstens dem Calciumsalz zu beschichten.

Unter Reiben des Körpers wird vorzugsweise ein mechanisches Anpressen des Körpers relativ zu der zu bcoohichtonden Oberfläche des medizinischen Implantats unter gleichzeitigen entgegengesetzten Bewegungen des Körpers entlang der zu beschichtenden Oberfläche des medizinischen Implantats verstanden. Beim Reiben kommt es zu einem Abtrag des wenigstens auf der Oberfläche des Körpers enthaltenen Calciumsalzes auf der zu beschichtenden Oberfläche des medizinischen Implantats. Daneben werden auch die weiteren optional enthaltenen Inhaltsstoffe des Körpers auf der zu beschichtenden Oberfläche des medizinischen Implantats abgetragen. Überraschenderweise wurde festgestellt, dass das vom Körper abgetragene Material hinreichend stabil auf der Oberfläche des medizinischen Implantats befestigt ist, um ein sicheres Einbringen des medizinischen Implantats in den Körper sicherzustellen.

Erfindungsgemäß ist somit die Verwendung eines Körpers, der wenigstens an einer seiner Oberflächen ein Calciumsalz mit einer Härte nach Mohs von nicht mehr als 5,5 aufweist, zum Aufbringen einer Beschichtung, die wenigstens dieses Calciumsalzes enthält, auf ein medizinisches Implantat durch Reiben.

Der Körper ist gemäß einer bevorzugten Ausführungsform Teil eines Stiftes, der zur Beschichtung des medizinischen Implantats dienen kann. Dieser Stift weist (i) einen Hohlkörper und (ii) einen in dem Hohlkörper bewegbar und aus dem Hohlkörper wenigstens teilweise ausführbar angeordneten Körper auf, der ein Calciumsalz mit einer Härte nach Mohs von nicht mehr als 5,5 und einen pharmazeutischen Wirkstoff umfasst.

Bei dem Körper des Stiftes handelt es sich vorzugsweise um einen Körper wie er hierin beschrieben ist. Dieser Körper weist erfindungsgemäß einen pharmazeutischen Wirkstoff auf. Vorzugsweise weist der Stift die Form eines Zylinders auf. Unter dem Begriff Zylinder wird vorzugsweise ein sphärischer Körper verstanden, der von zwei parallelen, ebenen Flächen (Grund- und Deckfläche) und einer Mantel- bzw. Zylinderfläche, die von parallelen Geraden gebildet wird, begrenzt ist. Ebenfalls fallen unter diesen Ragriff Körper, bei denen die Grundflächen und/oder die Deckflächen nicht plan, sondern abgerundet oder spitz zulaufend sind. Der Zylinder hat vorzugsweise einen Durchmesser von 5 - 25 mm und mehr bevorzugt einen Durchmesser von 10 - 15 mm. Mit zylindrischen Körpern, die diesen Durchmesser aufweisen, ist es möglich, auch schwerer zugängliche Oberflächenbereiche von medizinischen Implantaten zu erreichen und zu beschichten.

Der erfindungsgemäße Stift enthält einen Hohlkörper. Unter Hohlkörper wird dabei ein Körper verstanden, der einen Hohlraum aufweist. Der Hohlkörper ist so ausgestaltet, dass er den erfindungsgemäßen Körper wenigstens teilweise beherbergen kann.

Der Körper des Stiftes ist wenigstens teilweise in dem Hohlkörper untergebracht. Der Körper ist in dem Hohlraum des Hohlkörpers so angeordnet, dass er in dem Hohlkörper bewegbar ist. Vorzugsweise ist der Körper Im Hohlkörper entlang der Achse des Hohlkörpers bewegbar. Der Körper des Stiftes ist in dem Hohlkörper ferner so angeordnet, dass der Körper aus dem Hohlraum wenigstens teilweise ausführbar ist.

Vorzugsweise sind an dem Hohlkörper, an dem Körper oder an dem Hohlkörper und an dem Körper Mittel angebracht, die das Ausführen von wenigstens einem Teil des Körpers aus dem Hohlkörper durch Drückbewegungen oder Drehbewegungen erlauben.

Gemäß einer bevorzugten Ausführungsform sind an dem Hohlkörper, an dem Körper oder an dem Hohlkörper und an dem Körper Mittel angebracht, die eine Arretierung des Körpers in einer Position relativ zu dem Hohlkörper ermöglichen.

Ferner kann es bevorzugt sein, dass der Hohlkörper Mittel enthält, die ein einfaches Greifen des Hohlkörpers durch den Anwender ermöglichen. Zudem kann es bevorzugt sein, dass an dem Hohlkörper Mittel vorhanden sind, die ein Abrutschen der Hand des Anwenders in Richtung des Körpers verhindern.

Demnach ist es bevorzugt, dass wenigstens zwei und vorzugsweise vier Stege senkrecht an der Außenseite des Hohlkörpers angebracht sind, wobei jeweils zwei Stege zueinander auf einer Achse liegen, die die Achse des Hohlkörpers senkrecht schneidet. Vorzugsweise sind zwei gegenüberliegende Stege direkt an der Öffnung des Hohlkörpers angebracht, an der der Zylinder aus dem Hohlkörper tritt, um zu verhindern, dass der Anwender beim Herausdrücken des Zylinders mit seiner Hand bzw. den Handschuhen den Körper berühren kann. Durch diese Anordnung kann somit wirksam die Kontaminationsgefahr gesenkt werden. Die beiden anderen Otege können beim Horausdrücken des Körpers zum Festhalten des Hohlzylinders dienen. Gemäß einer bevorzugten Ausführungsform weist der erfindungsgemäße Stift einen Kolben auf. Der Kolben soll dazu dienen, den erfindungsgemäßen Körper aus dem Hohlkörper herauszuführen. Dieser Kolben ist demnach wenigstens teilweise in dem Hohlkörper angeordnet. Vorzugsweise ist der im Hohlkörper angeordnete Teil des Kolbens in dem Hohlkörper verschiebbar angeordnet. Dabei ist es bevorzugt, dass der im Hohlkörper angeordnete Teil des Kolbens entlang der Achse des Hohlzylinders verschiebbar ist. Ferner ist der Kolben vorzugsweise mit dem Hohlkörper verbunden. Bei dieser Verbindung kann es sich um eine starre oder lösbare Verbindung handeln. Beispielsweise kann der Kolben formschlüssig oder kraftschlüssig mit dem Hohlkörper verbunden sein. Gemäß einer bevorzugten Ausführungsform ist ferner die Summe der Längen des im Kolbens und des Hohlkörpers größer als die Länge des Hohlzylinders.

An einer Stirnseite des Kolbens können ferner ein oder mehrere Befestigungsstifte angeordnet sein. Diese Befestigungsstifte können zum Fixieren des Körpers am Kolben dienen. An der entgegengesetzten Stirnseite des Kolbens ist vorzugsweise ein Griff angeordnet. Dieser Griff kann im Zuge der Anwendung des erfindungsgemäßen Stiftes vom Operateur gegriffen werden und liegt demnach beim Herausdrücken des Hohlkörpers in der Handfläche des Operateurs. Beim Herausdrücken werden die mit wenigstens zwei Fingern umfassten Stege des Stiftes gegen den auf der Handfläche liegenden Griff gedrückt. Dadurch kann sich der Kolben nach vorne in Richtung der Öffnung des Hohlkörpers bewegen.

Gemäß einer weiteren bevorzugten Ausführungsform ist am Hohlkörper, am Kolben oder am Hohlkörper und am Kolben wenigstens eine Rastvorrichtung für den Körper angeordnet. Vorzugsweise sind als Rastung für den Körper am Hohlkörper bewegliche Rastungsstege angeordnet, die in ihrer Ruheposition in Richtung der Achse des Körpers zeigen. Durch die Rastung wird verhindert, dass der Kolben beim Reiben des Körpers auf die Oberfläche des medizinischen Implantats aus dem Hohlkörper fällt.

Ferner kann es bevorzugt sein, dass als Rastung auf dem Kolben Zähne oder umlaufende Befestigungsstege und im Hohlkörper Aussparungen angeordnet sind oder dass als Rastung auf dem Hohlkörper Befestigungsstege oder Aussparungen und im Kolben Aussparungen angeordnet sind.

Die Erfindung wird durch die nachstehenden Bespiele erläutert, die jedoch die Erfindung nicht beschränken.

### Beispiel 1:

Es wurde ein Gemisch von 71,7 Gewichtsprozent Calciumsulfat-Dihydrat, 17,9 Gewichtsprozent Calciumcarbonat, 8,8 Gewichtsprozent Glycerintripalmitat und 1,6 Gewichtsprozent Gentamicinsulfat mit einer Exzenter-Presse zu einem Zylinder mit einem Durchmesser von 22 mm und einer Höhe von 10 mm gepresst. Der Zylinder wurde auf beide Seiten einer gesandstrahlten Titan-Scheibe mit einem Durchmesser von 27 mm gerieben. Beim Reiben bildete sich eine dichte, farblose Schicht. Die Beschichtung wies eine Masse von 124,7 mg auf. Die Scheibe wurde in 5 ml 0,1 M Phosphat-Puffer bei 37 °C gelagert. Nach einem Tag wurde der Puffer vollständig entfernt und durch neuen Puffer ersetzt. Am zweiten Tag sowie am siebten Tag wurde der Puffer nochmals ausgetauscht. Die jeweils entnommene Puffer-Lösung wurde auf ihren Gentamicingehalt mit einem TDX-Analyzer der Firma Abbott untersucht.

| **Tag** | **Freigesetztes Gentamicin pro Quadratzentimeter der Oberfläche der Titanscheibe [µg/cm²]** |
|---|---|
| 1 | 92 |
| 2 | 18 |
| 6 | 1 |

### Beispiel 2:

Es wurde ein Gemisch von 61,7 Gewichtsprozent Calciumsulfat-Dihydrat, 10,0 Gewichtsprozent Strontiumsulfat, 17,9 Gewichtsprozent Calciumcarbonat, 8,8 Gewichtsprozent Glycerintripalmitat und 1,6 Gewichtsprozent Gentamicinsulfat mit einer Exzenter-Presse zu einem Zylinder mit einem Durchmesser von 15 mm und einer I löche von 30 mm geprcoot. Dor Zylinder wurde auf beide Seiten einer gesandstrahlten Titan-Scheibe mit einem Durchmesser von 27 mm gerieben. Beim Reiben bildete sich eine dichte, farblose Schicht. Die Beschichtung wies eine Masse von 111,2 mg auf.

### Beispiel 3:

Es wurde ein Gemisch von 61,7 Gewichtsprozent Calciumsulfat-Dihydrat, 10,0 Gewichtsprozent Strontiumsulfat,17,9 Gewichtsprozent Calciumcarbonat, 8,8 Gewichtsprozent Glycerintripalmitat und 1,6 Gewichtsprozent Gentamicinsulfat mit einer Exzenter-Presse zu einem Zylinder mit einem Durchmesser von 15 mm und einer Höhe von 30 mm gepresst. Der Zylinder wurde auf beide Seiten einer gesandstrahlten Titan-Scheibe mit einem Durchmesser von 27 mm gerieben. Beim Reiben bildete sich eine dichte, farblose Schicht. Die Beschichtung wies eine Masse von 111.2 mg auf.

### Beispiel 4:

Es wurde ein Gemisch von 71,7 Gewichtsprozent Calciumsulfat-Dihydrat, 17,9 Gewichtsprozent Calciumcarbonat, 8,8 Gewichtsprozent Glycerintripalmitat und 1,6 Gewichtsprozent Gentamicinsulfat mit einer Exzenter-Presse zu einem Zylinder mit einem Durchmesser von 15 mm und einer Höhe von 30 mm gepresst. Der Zylinder wurde auf beide Seiten einer gesandstrahlten Titan-Ocheibe mit einem Durchmesser von 27 mm gerieben. Beim Reiben bildete sich eine dichte, farblose Schicht. Die Beschichtung wies eine Masse von 112,1 mg auf.

### Beispiel 5:

Es wurde ein Gemisch von 71,9 Gewichtsprozent Calciumsulfat-Dihydrat, 17,9 Gewichtsprozent Calciumcarbonat, 8,8 Gewichtsprozent Glycerintripalmitat und 1,4 Gewichtsprozent Amikacinsulfat mit einer Exzenter-Presse zu einem Zylinder mit einem Durchmesser von 15 mm und einer Höhe von 30 mm gepresst. Der Zylinder wurde auf beide Seiten einer gesandstrahlten Titan-Scheibe mit einem Durchmesser von 27 mm gerieben. Beim Reiben bildete sich eine dichte, farblose Schicht. Die Beschichtung wies eine Masse von 110,4 mg auf.

### Beispiel 6:

Es wurde ein Gemisch von 72,3 Gewichtsprozent Calciumsulfat-Dihydrat, 17,9 Gewichtsprozent Calciumcarbonat, 8,8 Gewichtsprozent Glycerintripalmitat und 1,0 Gewichtsprozent Vancomycinhydrochlorid mit einer Exzenter-Presse zu einem Zylinder mit einem Durchmesser von 15 mm und einer Höhe von 30 mm gepresst. Der Zylinder wurde auf beide Seiten einer gesandstrahlten Titan-Scheibe mit einem Durchmesser von 27 mm gerieben. Beim Reiben bildete sich eine dichte, farblose Schicht. Die Beschichtung wies eine Masse von 117,1 mg auf.

### Beispiel 7:

Es wurde ein Gemisch von 70,0 Gewichtsprozent Calciumsulfat-Dihydrat, 18,6 Gewichtsprozent Calciumcarbonat, 10,0 Gewichtsprozent Polyethylenglykol 1000 und 1,4 Gewichtsprozent Amikacinsulfat mit einer Exzenter-Presse zu einem Zylinder mit einem Durchmesser von 15 mm und einer Höhe von 30 mm gepresst. Der Zylinder wurde auf beide Seiten einer gesandstrahlten Titan-Scheibe mit einem Durchmesser von 27 mm gerieben. Beim Reiben bildete sich eine dichte, farblose Schicht. Die Beschichtung wies eine Masse von 110,4 mg auf.

### Beispiel 8:

Es wurde ein Gemisch von 71,9 Gewichtsprozent Calciumsulfat-Dihydrat, 17,9 Gewichtsprozent Calciumcarbonat, 8,8 Gewichtsprozent Palmitinsäure und 1,4 Gewichtsprozent Amikacinsulfat mit einer Exzenter-Presse zu einem Zylinder mit einem Durchmesser von 15 mm und einer Höhe von 30 mm gepresst. Der Zylinder wurde auf beide Seiten einer gesandstrahlten Titan-Scheibe mit einem Durchmesser von 27 mm gerieben. Beim Reiben bildete sich eine dichte, farblose Schicht. Die Beschichtung wies eine Masse von 113,2 mg auf.

### Beispiel 9:

Es wurde ein Gemisch aus 60 Gewichtsprozent Calciumsulfat-Hemihydrat, 10 Gewichtsprozent Calciumcarbonat, 1,6 Gewichtsprozent Gentamicinsulfat und 28,4 Gewichtsprozent intensiv vermischt. Es entstand ein farblose, streichbare Masse, die in eine Silikonform gefüllt wurde. Die Silikonform hatte einen inneren zylinderförmigen Hohlraum mit einem Durchmesser von 15 mm und einer Länge von 40 mm. Die Masse härtete nach rund 15 Minuten aus. Danach wurde der Zylinder entnommen und getrocknet. Der getrocknete Zylinder wurde auf eine gesandtrahlte Titan-Scheibe mit einem Durchmesser von 27 mm gerieben. Die gebildete Beschichtung wies eine Masse von 128,2 mg auf.

## Patentansprüche

1. Verfahren zur Beschichtung eines medizinischen Implantats, bei dem man
(i) ein medizinisches Implantat bereitstellt, das wenigstens eine zu beschichtende Oberfläche aufweist.
(ii) einen Körper, der wenigstens an einer seiner Oberflächen wenigstens ein Calciumsalz mit einer Härte nach Mohs von nicht mehr als 5,5 aufweist, bereitstellt, und
(iii) den Körper mit der das Calciumsalz enthaltenden Oberfläche auf die wenigstens eine zu beschichtende Oberfläche des medizinischen Implantats reibt, um diese Oberfläche des medizinischen Implantats mit wenigstens dem Calciumsalz zu beschichten.

2. Verfahren nach Anspruch 1, **dadurch gekennzeichnet, dass** es sich bei dem medizinischen Implantat um eine Gelenkendoprothese handelt.

3. Verfahren nach Anspruch 1 oder 2, **dadurch gekennzeichnet, dass** die zu beschichtende Oberfläche des medizinischen Implantats eine mittlere Rauheit Rₐ von wenigstens 0,5 µm aufweist.

4. Verfahren nach einem der Ansprüche 1-3, **dadurch gekennzeichnet, dass** die zu beschichtende Oberfläche des medizinischen Implantats eine Härte nach Mohs aufweist, die größer ist als die Härte nach Mohs der das Calciumsalz aufweisenden Oberfläche des Körpers.

5. Verfahren nach einem der Ansprüche 1 - 4, **dadurch gekennzeichnet, dass** der Körper eine Porosität im Bereich von 0 - 70 Volumenprozent aufweist.

6. Verfahren nach einem der Ansprüche 1 - 5, **dadurch gekennzeichnet, dass** der Körper einen Wasseranteil im Bereich von 0-70 Gewichtsprozent aufweist.

7. Verfahren nach einem der Ansprüche 1 -6, **dadurch gekennzeichnet, dass** das wenigstens eine Calciumsalz eine Löslichkeit in Wasser bei einer Temperatur von 25°C von wenigstens 2 g/l aufweist.

8. Verfahren nach einem der Ansprüche 1 - 7, **dadurch gekennzeichnet, dass** der Körper 50-100 Gewichtsprozent Calciumsalze enthält.

9. Verfahren nach einem der Ansprüche 1 - 8, **dadurch gekennzeichnet, dass** das Calciumsalz ein anorganisches Calciumsalz ist.

10. Verfahren nach einem der Ansprüche 1-9, **dadurch gekennzeichnet, dass** das Calciumsalz aus der Gruppe ausgewählt ist, die aus Calciumsulfaten, Calciumphosphaten, Calciumhydrogenphosphaten und Calciumcarbonat besteht.

11. Verfahren nach Anspruch 10, **dadurch gekennzeichnet, dass** das Calciumsulfat aus der Gruppe ausgewählt ist, die aus kristallwasserhaltigem Calciumsulfat und kristallwasserfreiem Calciumsulfat besteht.

12. Verfahren nach Anspruch 11, **dadurch gekennzeichnet, dass** das kristallwasserhaltige Calciumsulfat aus der Gruppe ausgewählt ist, die aus Calciumsulfat-Dihydrat und Calciumsulfat-Hemihydrat besteht.

13. Verfahren nach einem der Ansprüche 1-12, **dadurch gekennzeichnet, dass** der Körper eine Verbindung aufweist, die aus der Gruppe ausgewählt ist, die aus Fettsäuren, Fettsäureestern, Fettsäuresalzen, Polyethylenglykolen mit einer Molmasse von nicht mehr als 1000 g/mol und oligomeren Milchsäureestern mit einer Molmasse von weniger als 1000 g/mol besteht.

14. Verfahren nach einem der Ansprüche 1-13, **dadurch gekennzeichnet, dass** der Körper wenigstens einen pharmazeutischen Wirkstoff enthält.

15. Verfahren nach einem der Ansprüche 1 - 14, **dadurch gekennzeichnet, dass** der Körper wenigstens einen Farbstoff enthält.

16. Verfahren nach einem der Ansprüche 1-15, **dadurch gekennzeichnet, dass** der Körper ein Salz enthält, das aus der Gruppe ausgewählt ist, die aus Magnesiumsalzen, Strontiumsalzen und Lithiumsalzen besteht.

17. Verfahren nach einem der Ansprüche 1-16, **dadurch gekennzeichnet, dass** der Körper 70 - 90 Gewichtsprozent Calciumsulfat und 10-30 Gewichtsprozent Calciumcarbonat enthält.

18. Verfahren nach einem der Ansprüche 1 16, **dadurch gekennzeichnet, dass** der Körper 40 - 90 Gewichtsprozent Calciumsulfat, 0,1-40 Gewichtsprozent Calciumcarbonat und 0,1 - 20 Gewichtsprozent Strontiumsulfat enthält.

19. Verfahren nach einem der Ansprüche 1-16, **dadurch gekennzeichnet, dass** der Körper 20 - 90 Gewichtsprozent Calciumsulfat, 0,1-40 Gewichtsprozent Calciumcarbonat und 0,5-50 Gewichtsprozent eines pharmazeutischen Wirkstoffs enthält.

20. Verfahren nach einem der Ansprüche 1-17, **dadurch gekennzeichnet, dass** der Körper 70 - 73 Gewichtsprozent Calciumsulfat, 16 - 20 Gewichtsprozent Calciumcarbonat, 8-10 Gewichtsprozent Glycerintripalmitat und 1- 2 Gewichtsprozent Gentamicinsulfat enthält.

21. Verwendung eines Körpers, der wenigstens an einer seiner Oberflächen ein Calciumsalz mit einer Härte nach Mohs von nicht mehr als 5,5 aufweist, zum Aufbringen einer Beschichtung, die wenigstens dieses Calciumsalz enthält, auf ein medizinisches Implantat durch Reiben.

22. Stift zur Beschichtung eines medizinischen Implantats aufweisend einen Hohlkörper und einen in dem Hohlkörper bewegbar und aus dem Hohlkörper wenigstens teilweise ausführbar angeordneten Körper, der ein Calciumsalz mit einer Härte nach Mohs von nicht mehr als 5,5 und einen pharmazeutischen Wirkstoff umfasst.
